(19) Europäisches Patentamt
European Patent Office
Office européen des brevets

(11) Publication number: **0 443 335 A1**

# (12) EUROPEAN PATENT APPLICATION

(21) Application number: **91100773.0**

(22) Date of filing: **22.01.91**

(51) Int. Cl.[5]: **C12N 15/86**, C12N 5/10, C12P 21/02

The title of the invention has been amended (Guidelines for Examination in the EPO, A-III, 7.3).

(30) Priority: **29.01.90 DE 4002521**

(43) Date of publication of application: **28.08.91 Bulletin 91/35**

(84) Designated Contracting States: **AT BE CH DE DK ES FR GB IT LI LU NL SE**

(71) Applicant: **IMMUNO Aktiengesellschaft**
**Industriestrasse 67**
**A-1221 Wien(AT)**

(72) Inventor: **Bodemer, Walter, Dr. Dipl.-bio.**
**Hessegasse 32/31**
**A-1228 Wien(AT)**
Inventor: **Scheiflinger, Friedrich, Dipl.-Ing.**
**Raiffeisenstrasse 15**
**A-2304 Orth/Donau(AT)**

(74) Representative: **Patentanwälte Grünecker, Kinkeldey, Stockmair & Partner**
**Maximilianstrasse 58**
**W-8000 München 22(DE)**

## (54) Recombinant pox virus plasmid containing a multi-functional DNA cassette.

(57) The invention relates to a recombinant plasmid containing at least one replicon (oril), at least one selection marker (sm1), at least one promoter (pr1) and DNA sequences from a pox virus genome flanking this promoter, a multi-functional DNA sequence cassette being arranged downstream of this promoter (pr1). This multi-functional DNA sequence oassette contains sequences which are complementary to commercially available primer sequences which proved their worth in practice, a translation initiation codon and several restriction endonuclese cleavage sites and stop codons in all three frames following this translation initiation codon.

The recombinant plasmid according to the invention is excellently suited for the expression of foreign proteins in the cytoplasma of eukaryontic host cells after insertion into a pox virus genome.

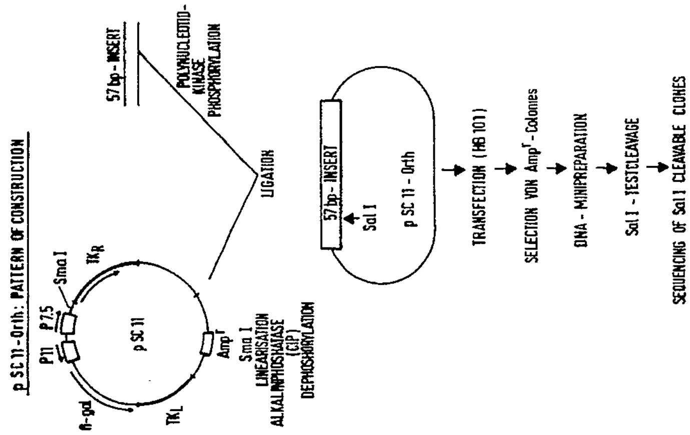


FIG. 2

EP 0 443 335 A1

## A RECOMBINANT PLASMID

The invention relates to a recombinant plasmid, containing at least one replicon (oril), at least one selection marker (sml), at least one promoter (prl) and DNA sequences from a pox virus genome flanking this promoter, a process for the production of a recombinant pox virus, a recombinant pox virus, the use of the recombinant pox virus, a cell line, a peptide or polypeptide, a composition, a vaccine and a virus-vaccine, a diagnostic agent and the use of the vaccines.

By means of the development of modern genetic engineering methods it has been possible to identify and isolate a respectable number of genes for proteins being diagnostically, prophylactically and therapeutically important, e.g. the genes for coagulation factors or antigenic virus proteins. In connection with this, the need of reliable and efficient expression systems in procaryotic or eucaryotic cells increases in order to be able to isolate the desired proteins either from infected tissue or infected cell culture or e.g. to use the immunogenic properties of proteins expressed in vivo for immunization. Eucaryotic viral vector systems have gained special importance here.

One of the known eucaryotic vector systems is the papova virus SV40. SV40 has a comparatively small genome with 5.3 kb, whose organization is relatively well known. The small genome of this virus has certainly, on the one hand, the advantage that it can be manipulated in targeted fashion if suited restriction endonucleases are used, however, on the other hand, it has the disadvantage that the cloning capacity is greatly restricted by a correspondingly small virus envelope and by the compact partly overlapping arrangement of essential genes.

The development of a eucaryotic vector systems on the basis of the vaccinia virus was described in more recent works (e.g. EP-A-0243029). The vaccinia virus is an orthopox virus having a linear genome of double-stranded DNA of about 185 kb. The size of the vacinia virus genome makes the construction of recombinant genomes by cleavage with restriction endonucleases and subsequent ligation with suited foreign DNA impossible. Even if this process would be practicable, the production of viruses with the recombinant genome would require the presence of a helper virus, since the virus DNA manipulated in vitro itself is not infectious. The transcription of the vaccinia virus requires i.a. a vaccinia-virus-coded RNA polymerase which cannot be replaced by the eucaryotic RNA polymerase II and must be introduced together with the infection, delivered from virus particles. Accordingly. the vaccinia virus has special promoters recognized by the virus-coded RNA polymerase. This means a further difficulty in the construction of recombinants which are to express foreign genes inserted into the vaccinia virus genome in eucaryotic cells. The use of vaccinia viruses as expression vectors thus requires that the transcription and replication can be carried out by vaccinia-inherent enzymes independently of the host, i.e. that, on the one hand, no essential virus regions may be affected by the cloning and that, on the other hand, genes provided for expression must be under the control of vaccinia promoters.

For this reason a process comprising two steps is used in general, in which first a plasmid is constructed which contains the desired foreign gene in combination with a vaccinia virus promoter and this recombinant plasmid is inserted in vivo into the vaccinia virus genome by means of homologous recombination between virus and plasmid DNA. A recombinant vaccinia virus is e.g. described in the EP-A-0243029, which has been constructed according to the principle described above and is used for the expression of the HIV-1 env protein. The plasmid pSC25 used in said European patent application is a vector which is extremely suited for the intended expression of the env protein.

However, both plasmids, pSC25 and the parental plasmid pSC11, are completely unsuited for further development, i.e. for insertion of further and other foreign DNA. pSC25, on the one hand, has no further acceptable cloning site, and in the precursor plasmid pSC11, on the other hand, just a single SmaI site is indicated.

A cloning into a SmaI site requires in most cases a manipulation of the restriction fragments which are inserted into the site, since SmaI is a restriction enzyme producing blunt ends. It is moreover not possible to clone gene fragments in the plasmid pSC11, which do not contain any translation signals of their own, i.e. a start or stop codon.

Although the plasmid pSC11 shows many advantages for the cloning of incomplete genes, gene fragments or even synthetic DNA, its use requires extensive and troublesome construction processes.

Therefore it was the object of the invention to make a plasmid available which facilitates the cloning of foreign DNA in a plasmid capable of recombination with the pox virus inasmuch as this is possible.

This object is accomplished by a recombinant plasmid according to the preamble of the main claim by arranging a multi-functional DNA sequence cassette downstream of the promoter (prl).

The recombinant plasmid according to the invention was a multi-functional DNA sequence cassette

arranged downstream of the promoter. This construction offers the advantage that different functional sequences can be inserted according to any set problem. It is e.g. conceivable to use sequence cassettes which use the start codon GUG, which is used less frequently in addition to the translation start codon AUG which occurs most frequently. It is equally possible to use cassettes with one or several stop codons which may be the same or different to prevent any possibly occurring amber, ochre or opal suppression. The systems permits moreover the insertion of polyadenylation signals and other signal structures in each case in addition to the insertion of the desired foreign gene or gene fragment.

The plasmid according to the invention contains in its multi-functional DNA sequence cassette preferably multiple cloning sites. Customary cloning sites for hexamer recognizing rectriction endonucleases are preferred here which produce sticky ends. The use of such restriction endonuclease cleavage sites makes the targeted cloning of DNA fragments from the genome of every desired organism possible. The restriction endonuclease cleavage sites contained in the multi-functional DNA sequence cassettes are preferably selected in such fashion that they are cleavage sites for commercially available restriction endonucleases, whose use is also recommendable under economic view-points.

Examples for such restriction endonuclease cleavage sites are the cleavage sites for the enzymes Stel, Avrll, Sall, Accl, Hindll, Spel, Nhel. However, cleavage sites for all known restriction endonucleases can be used according to the invention. In this connection reference is made to the extensive enumeration of known restriction endonucleases by Kessler, C. and Höltke, H-J (Gene 47, pages 1 et sequ. 1986). The usability of individual restriction endonucleases is at best restricted by the fact that further cleavage sites are already present for this very restriction endonuclease in the plasmid according to the invention. In this case a person skilled in the art will decide whether he either switches to an alternative enzyme or changes the plasmid by site-specific mutagenesis in such fashion that it no longer has the corresponding restriction endonuclease cleavage site or uses the partially cleaved DNA for ligation.

In a preferred embodiment the multi-functional DNA sequence cassette provides translation start codons and translation stop codons. Appropriately, these codons are disposed in such fashion that they flank a number of customary restriction endonuclease recognition sites. This means for instance that the sequence ATG is inserted in such fashion in the 5' region of the restriction endonuclease cleavage sites that a cloning in the subsequent cleavage sites in several different frames is possible. Following the region containing the restriction endonuclease cleavage sites, several translation stop codons should be disposed, namely also in such fashion that they terminate a translation possible in all conceivable frames. It is preferred that different stop codons succeed each other to ensure the translation termination a 100%.

According to the invention a promoter (pr1) is already made available by the multi-functional DNA sequence cassette. There is moreover the possibility of inserting a further promoter (pr2) into one of the cloning sites, preferably a restriction endonuclease cleavage site disposed in the 5' region of the sequence cassette. It offers itself to use promoters which can for instance be induced or have, in other respect, an advantage over the already existing promoter (pr1).

The multi-functional DNA sequence cassette can moreover be constructed in such fashion that it contains enhancing DNA sequences in the 5' or 3' region of the mcs (multiple cloning sites). So far, such enhancing sequences have been known above all from viruses, e.g. from SV40, from the polyoma virus, the papova virus and others. sequences are concerned which are capable of replacing e.g. the function of the 72-base-pair-long repetition from SV40 DNA. DNA sequences with enhancer function identified so far have a length of from only a few base pairs to 72 base pairs.

As a function of the nature of a cloned gene to be expressed and the conditions connected therewith, e.g. expression rate, cell toxicity and the like, it is advisable to provide the proteins with hydrophobic membrane anchors or signal peptides. Accordingly DNA sequences can be incorporated into the multi-functional DNA sequence cassette, which can either code for the hydrophobic membrane anchor or for a desired signal peptide which consists mostly of a hydrophilic N-terminal region and a subsequent hydrophobic share. A person skilled in the art knows a number possible usable signal peptides for this from the literature.

In a preferred embodiment the multi-functional DNA sequence cassette contains sequences at its 5' and/or 3' end which are complementary to known oligonucleotide primers. Generally customary oligonucleotides for sequencing can e.g. be concerned.

In a preferred embodiment the sequences with complementarity to known primers disposed on the 5' and/or 3' end correspond to such sequences which are e.g. recognized by the SP6 polymerase or the T7 polymerase. Known primers which link for instance to the Sp6 or T7 promoter region are 5'-CACATACGATTTAGG-3' (15-mer) and/or 5'-ATCGAAATTAATACG-3' (15-mer). The making available of sequences being complementary to such oligonucleotides offers the advantage that the sequences of the inserted DNA fragment including the foreign gene fragment can be checked at any time in a manner known

per se with double-strand sequencing methods.

In a preferred embodiment the plasmid according to the invention contains the following multi-functional DNA sequence cassette:

**5'-GCACATACGATTTAGGCCTAGGATGTCGACTAGTTAGCTAGCGTATTAATT-TCGATC-3'**

The function of the nucleotides is explained in greater detail in Fig. 1.

The construction of the plasmid according to the invention is preferably carried out in customarily used microorganisms, e.g. in procaryotic organisms, preferably in gram-negative bacteria. For this reason, the plasmid according to the invention contains sequence regions which are necessary for replication and amplification in respectively desired procaryontes e.g. a replication origin in addition to its functions necessary for recombination in the cytoplasma of a eucaryotic tic cell. A replicon making the replication of a plasmid in the procaryot: possible is customarily used as oriI. It is preferred for the construction of the recombinant plasmid that transformation and amplification of the recombinant plasmid are carried out in gram-negative bacteria, e.g. in Escherichia coli. Therefore the Col E 1 replicon from Escherichia coli is an especially preferred replicon.

The various steps necessary for the contruction or amplification of the recombinant plasmid can, however, also be carried out in eucaryontes. In this case yeast must be indicated as preferred organism; yeasts are well characterized eukaryontic organisms which are comparatively easy to handle. In this case, a replicon to be used with preference would be e.g. the replicon of the $2\mu$ plasmid from Saccharomyces.

The steps necessary for the construction of the plasmid according to the invention, e.g. the selection of transformed bacteria is facilitated by the use of a suited selection marker. A preferred selection marker in the transformation of procaryontes e.g. the inactivation of antibiotics. A person skilled in the art knows a plurality of resistance genes for the expression of antibiotics-inactivating proteins in gram-negative and gram-positive organisms.

If, on the other hand, the construction of the recombinant plasmid is carried out in the eucaryotes, e.g. yeast, genes from the amino acid or nucleic acid metabolism of yeast can be used as customary selection markers. In the case of the use of appropriate yeast mutants selection can be carried out with respect to such transformed yeast which are capable of expressing the metabolism gene deficient in the transformed strain. Isolated yeast genes may likewise serve for complementation of amino acid metabolism mutants in several eucaryotic organisms, e.g. primitive algae, Physarum or Dictyostelium.

In preferred embodiments of the invention an especially strong promoter is used as promoter pr1 to intensify the expression of the desired foreign gene. It is necessary to use a promoter which is recognized by the pox RNA polymerase in the pox system. As already mentioned, the transcription of pox genes takes place in the cytoplasma of the eucaryotic cell and is carried out exclusively by pox-inherent RNA polymerases.

An especially preferred promoter is the vaccinia promoter p7.5, which is responsible for a polypeptide with a molecular weight of 7.5 kD in the wild-type vaccinia virus. This promoter has the particularity that the expression of the gene controlled by it takes place at almost each time of the vaccinia replication cycle. The promoter p7.5 is the single characterized promoter, of which it is known at present that it makes a uniform expression possible over the entire life cycle of vaccinia.

In further preferred embodiments the promoter p7.5 is replaced by chemically synthetized promoters or by promoters changed by mutagenesis, which can also be recognized by the pox-inherent RNA polymerase. The promoter p7.5 can e.g. be changed in a manner known to a person skilled in the art by exchanging individual bases, the effectivness of the changed promoter can be tested in vivo and such a changed promoter can possibly be incorporated into the plasmid according to the invention.

The integrity of the virus genome must not be destroyed by the recombination of the plasmid according to the invention with the wild-type pox virus, i.e. the integration of foreign DNA following from the recombination must not desturb any essential virus gene. It is already established to use a DNA region from the thymidine kinase gene of the vaccinia virus as the sequence serving for recombination. The thymidine kinase gene is a gene present in the wild-type virus, but not essential at all in normal eucaryotic host cells, which is already isolated. If two fragments of this gene are used as flanking regions for a DNA fragment intended for the insertion into the pox virus, one finds this DNA fragment at the corresponding sites of the virus thymidine kinase gene after recombination.

The use of the thymidine kinase gene has the special advantage that in the case of the simultaneous

use of TK$^{\ominus}$ host cells a bromodesoxyuridine selection can be carried out after transformation with the plasmid according to the invention. Thus such cells can be identified in simple fashion in which the function of the thymidine kinase gene of the wild-type virus has been destroyed by recombination of the intracellularly present, intact vaccinia virus with the plasmid according to the invention.

It is moreover also possible to use DNA sequences from other, non-essential regions instead of non-essential regions of the pox virus genome from the thymidine kinase gene. The prerequisite for the use of a specific DNA region is only that the destruction of the corresponding viral copy must not impair the viability of the recombinant virus.

The plasmid according to the invention contains preferably a second selection marker (sm2) which serves for the identification of infected cells. In a preferred embodiment the lacZ gene is used as the second selection marker. The expression of the gene product of the lacZ gene, the $\beta$-galactosidase can be easily detected by means of the chromogenic substrate 5-bromo-4-chloro-3-indolyl-$\beta$-D-galactopyranoside (x-gal) so that cells transformed with the plasmid according to the invention can be identified relatively easily in a cell culture.

The expression of the second selection marker must also be under the control of a pox virus promoter. Any other promoter functioning in the pox virus system can of course also be used.

A promoter used with preference for these purposes is the "late" promoter p11, which codes for a polypeptide with a molecular weight of 11 kD in the wild-type vaccinia virus. P11 is also a strong promoter which brings about the expression of a sufficient amount of $\beta$-galactosidase so that it is possible to detect the enzyme with the said chromogenic substrate.

An alternative to the use of the lacZ gene as sm2 is the use of a dominant selection marker. An example of such a dominant selection marker is e.g. the gpt gene which codes for the xanthine guanine phosphoribosyl transferase. This enzyme is a gene product from E. coli analogous to the hypoxanthine guanine phosphoribosyl transferase from mammalian cells. Like the mammalian cell enzyme, it catalyzes the condensation of phosphoribosyl pyrophosphate with hypoxanthine or guanine to inosine or guanylic acid (IMP and/or GMP). The bacterial enzyme has, however, moreover also the property of being able to phosphorylate xanthine to XMP, a reaction which the enzyme from mammalian cell cannot carry out or only carries out poorly. A recombinant virus which carries the gpt gene can therefore enable mammalian cells infected with this virus to synthesize GMP and thus dGTP and DNA by the provision of XMP in the presence of mycophenolic acid, while the synthesis of XMP by the mycophenolic acid is inhibited in non-infected cells, the utilization of the offered xanthine is, however, not possible due to the different specificity of the analogous mammalian cell enzyme.

An alternative for the expression of the gpt gene as a selection marker would be the use of the gene for the hygromycin-B-phosphotransferase. Hygromycin-B is an antibiotic synthetized from Streptomyces, which inhibits the protein synthesis both in the prokaryont and in the eucaryote by inhibiting the ribosomal translocation and thus interferes with the aminoacyl-t-RNA recognition. This antibiotic agent can be inactivated by a phosphorus transferase isolated from E. coli.

The plasmid made available according to the invention can be used for the cloning of complete genes. The redundancy of promoters, which occurs possibly with this, is insignificant inasmuch as only the pox-inherent promoter will be recognized in the cytoplasma of a cell infected with the pox virus inasmuch as it is known so far.

However, the plasmid according to the invention serves with preference for the cloning of sequences which do no longer contain any endogenous transcription or translation start signals. This applies e.g. to the targeted cloning of exclusively functional regions of a protein and/or the cloning of antigenic determinants. The cloning of such fragments gains increasing importance above all in connection with the production of vaccines. In specific cases, the replication function of the virus produced by means of recombination with the plasmid according to the invention can be reduced by the insertion of a complete gene or the cell may be prematurely damaged to too great an extent. In many cases only parts of the proteins are moreover only necessary for specific tasks.

The DNA inserted into the plasmid according to the invention can be both of natural and synthetic origin. The DNA of natural origin will preferably be either directly a genomic DNA e.g. isolated from the genome of procaryotes,, or cDNA of more complex, eucaryotic genes. A pecularity of the pox virus system is that so far it has not been possible to detect any splicing of mRNA. This is presumably attributable to the fact that all virus-dependent processes take place in the cytoplasma of the infected cell, while the cellular splicing apparatus is located in the nucleus. Therefore care must be taken in the construction of the plasmids which are to be used for recombination with the vaccinia virus that intron-free DNA is used in the case of the use of eucaryotic genes.

As a rule, synthetic DNA will be previously composed of oligonucleotides produced synthetically and

subsequently transferred into the plasmid according to the invention. The use of synthetic DNA has the advantage that it can already be modified in accordance with the later requirements during the production and that the restriction cleavage sites used later for the cloning can be incorporated at the termini. Moreover, a connection of synthetic and natural DNA is possible owing to the fact that a further cassette is inserted into the already existing mcs which contains the restriction endonuclease cleavage sites necessary for the special cloning problem.

Proteins to be cloned with preference are proteins or their derivatives from pathogens, which can e.g. be used for immunization purposes. Examples of this are pertussis, tetanus, malaria, AIDS, tick-borne encephalitis, hepatitis B and herpes infections. This enumeration is of course not exclusive. In most cases the proteins to be used for immunization will induce neutralizing antibodies and/or a T cell response.

This means in most cases, but not always, that surface proteins are concerned. Examples of this are offered by influenza, herpes and flavi viruses.

A further field for the use of the plasmid according to the invention for the production of recombinant pox viruses is the production of proteins with the biological functions of at least one of the following proteins: one of the coagulation factors II, VIII, IX, XII, XIII von Willebrand factor, protein C and S, antithrombin III, plasminogen, hirudin, NGF, FGF, TNF, B-cell stimulating factors, proteins from the interleukine group, apolipoproteins or glycoproteins or non-structural proteins from viruses. An essential advantage of the isolation of these proteins from the pox virus expression system is the fact that e.g. the coagulation factors which are conventionally isolated from blood sera will not be contaminated with other viruses, e.g. AIDS viruses. The isolation of these factors from the cell culture moreover makes a concentration on a large scale possible. The production of recombinant pox virus using the plasmid according to the invention is carried out in vivo by means of the recombination of the flanking pox virus sequences, i.e. preferably of the thymidine kinase gene sequences, of the plasmid with the pox virus. The originally intact viral gene is destroyed and thus inactivated by the recombination.

In a preferred embodiment the plasmid according to the invention is pSC11-Orth. pSC11-Orth is a derivative of the known plasmid pSC11; the pattern of its construction is shown in Fig. 2. pSC11-Orth was deposited with the DSM in Braunschweig on January 9, 1990 and received the deposition number DSM 5734.

The production of recombinant pox viruses is effected by infecting suitable cell line, if possible a $TK^-$ cell line which is infected with a functioning pox virus, e.g. a vaccinia virus adapted to laboratory requirements, in particular a vaccine strain. Preferred viruses are attentuated viruses, e.g. such strains whose hr (human host range) gene or VGF (vaccinia growth factor) gene is inactive due to complete or partial deletion or mutation. Examples of such viruses are described in Altenburger et al., Arch. Virol. 105, pages 15 to 27, 1989 and C. Kaplan, Arch. Virol. 106, pages 127 to 139, 1989.

Known processes with which a person skilled in the art is familiar are used for the transformation, e.g. calcium phosphate precipitation, liposome fusion, electroporation. A plasmid according to the invention which was transferred into the cell to be transformed will recombine in vivo with the already present virus genome without any further help by the experimentator. As already mentioned above, the sucessful transformation can be tested by the addition of X-gal, if sm2 is the gene for the $\beta$-galactosidase. The successful recombination must moreover be verified by a bromodesoxyuridine selection. Only recombinant $TK^-$ viruses survive in the $TK^-$ cells, because they cannot incorporate the offered bromodesoxyuridine into their DNA.

Recombinant pox viruses are formed by the recombination, which contain, in addition to the natural pox components, a foreign gene or parts thereof being under the control of an additional promoter as a function of the nature of the plasmid used according to the invention. As a function of the nature of the promoter, this foreign gene is transcribed either during the early or late transcription phase of the virus or over the entire transcription time.

The aforementioned pox viruses are vaccinia viruses in a preferred embodiment. The vaccinia virus system is already characterized very well and offers great advantages in view of the wealth of available knowledge.

The pox viruses acording to the invention can be used for the expression of DNA sequences foreign to the pox-virus as already mentioned. The pox virus system does not only offer the advantage of an efficient expression, but moreover the advantage, as opposed to the expression in procaryotes, that the formed products are correctly modified.

The formed transcripts are translated in the cytoplasma of the infected cell and subsequently post-translationally modified, i.e. e.g. glycosylated, carboxylated or acetylated. The system according to the invention can therefore be used for the expression of eukaryontic DNA sequences foreign to the pox-virus and leads to authentically modified proteins.

Proteins which can be produced with preference with this system are e.g., as already mentioned above, functional proteins from pathogens. The recombinant pox viruses code as a function of the plasmid used for recombination for proteins or their derivatives from pathogens or, however, also for parts of such proteins.

Important pathogens whose proteins can be produced in this fashion are e.g. pertussis, tetanus, malaria, AIDS, tick-borne encephalitis, hepatitis B and herpes.

The coagulation factors II, VIII, IX, XII, XIII, von Willebrand factor, protein C and S, antithrombin III, plasminogen, hirudin, NGF, FGF, TNF, B-cell stimulating factors, proteins from the interleukine group, apolipoproteins or glycoproteins or non-structural proteins, e.g. of the FSME virus can furthermore be produced with this system. The recombinant vaccinia viruses according to the invention can be kept in cell cultures. It is advisable for a lasting keeping to freeze or lyophilize the cell cultures infected with the viruses according to the invention.

A cell culture to be used with preference consists of $TK^-$ cells or Vero cells for the making available of larger amounts of viruses, e.g. cells of the line $TK^-143$ (CNCM; I-732).

The peptide or polypeptide coded by the foreign DNA is synthetized in the cell cultures according to the invention continuously or only at determined times of the virus cycle as a function of the used promoter. The peptides or polypeptides produced in this fashion can be isolated from the cells in a manner known to a person skilled in the art and purified according to customary processes of protein chemistry. A special advantage of the system is that proteins expressed under the control of the promoter p7.5 can be incorporated into the outer membrane , if they have anchor sequences or the like, and then they can thus already be recognized as antigens.

The peptides or polypeptides according to the invention are suited for the production of a composition which may contain optionally only one of the produced protein species, but also several ones, to produce thus e.g. a polyvalent vaccine. The composition may contain physiologically tolerable additives in addition to the peptides or polypeptides, whose nature will depend on the intended use of the composition. Possible additives are e.g. adjuvants, stabilizers, buffers or osmotically active substances.

The compositions according to the invention can then be used for the production of a vaccine. As a function of the nature of the peptide or polypeptide produced in the cells this vaccine can be intended for the application both in man and in animals, e.g. domestic animals and domestic cattle.

The composition according to the invention can furthermore be used as a diagnostic agent. In this case a detection of antibodies against specific proteins of a pathogenic virus in infected individuals would e. g. be possible by an antigen/antibody reaction between the peptide or polypeptide produced according to the invention and the serum of the human or animal patient.

The Figs. and the examples explain the invention:

Description of the Figs.

Fig. 1A shows the 57 bp-insert which is inserted into the vector PSC11 according to the invention. Various restriction endonuclease cleavage sites usable for the production of recombinant plasmids are indicated in the drawing and moreover the sequence regions which hybridize with an SP6 primer or T7 primer; the translation start codon contained in the 57 bp-insert and stop codons are marked in various frames.

Fig. 1B shows a sequence gel on which the sequence of the insert has been checked, on the one hand, using the SP6 primer (lefthand sample) and, on the other hand, using the T7 primer. The sequence gel shows clearly that the plasmid pSC11-Orth according to the invention contains the 57-base-pair sequence,

Fig. 2 shows the construction pattern on which the construction and identification of the plasmid pSC11-Orth has been based.

Fig. 3 illustrates the insertion of a DNA sequence described in example 2, which codes for part of the surface antigen of HIV-1. The HIV-1 sequences are constructed with the respective vector sequences by italics.

Fig. 4 illustrates the example 3, in which the insertion of a DNA sequence which codes for part of the surface antigen glycoprotein E of the FSME virus, is described. In the construction of a vector derived from pSC11 -Orth, which expresses the surface antigen, a synthetic adaptor had to be inserted in this case to maintain the translation frame. This adaptor is contrasted with the sequences of the plasmid according to the invention by bold type. The sequences originating from the TBE virus are represented in italics.

Example 1

Construction of the plasmid pSC11-Orth

In the construction of the pSC11 derivative pSC11-Orth a double-stranded sequence of 57 base pairs was inserted into the parental plasmid pSC11 (DSM 4381; Chakrabarti et al., Mol. and Cell. Biol. 5, pages 3404 to 3409, 1985), which contains several functional sequences. The procedure is represented in Fig. 2.

All steps are carried out according to processes with which a person skilled in the art is familiar and which are e.g. described in T. Maniatis et al., "Molecular Cloning", Cold Spring Harbor 1982.

Example 2

Insertion of a DNA sequence which codes for part of the surface antigen gp160 of the HIV-1

The DNA of the pSC11-Orth plasmid was linearized with the restriction endonuclease SalI. The projecting 5' ends in the pSC11-Orth formed after linearizing with the restriction endonuclease were degraded with S1 nuclease to receive a PvuII/BglII fragment (Hahn et al., Nature 312, pages 166 to 169, 1984) from the plasmid pBH10 (Ratner et al., Aids Res. and Human Retroviruses 3, pages 57 to 65, 1987) which codes for part of the surface antigen gp160. After this treatment the DNA was inserted for ligations with the HIV-specific PvuII/BglII fragment (536 bp). For this purpose the BglII end of the PvuII/BglII fragment was either treated with S1 nuclease or filled with Klenow DNA polymerase to obtain a blunt clonable 3' end (the 5' terminal PvuII cleavage site is already blunt). In each case the ATG originates from pSC11-Orth. If the Klenow polymerase is used for filling, amino acids are additionally obtained at the 3' end. The stop codons, in turn, originate from the pSC11-Orth; after filling of the BglII cleavage site the translation ends at stop 1 (cf. Fig. 1) after digestion of protruding single-stranded sequences with S1 nuclease at stop 3.

The correct orientation of the HIV insert was confirmed by means of the analysis with various restriction endonucleases.

Example 3

Insertion of a DNA sequence which codes for part of the surface antigen glycoprotein E of the TBE virus

The DNA of the pSC11-Orth plasmid was linearized with the restriction endonuclease SalI and dephosporylated. The sequence region coding for part of the glycoprotein E was cleaved out from the cDNA-clone pA5 (DSM 4382; Mandl et al., Virology 166, pages 197 to 205, 1988) of the TBE virus with the restriction endonuclease PvuII and the formed fragment was isolated from the agarose gel. Inserting this PvuII fragment into the SalI site of the plasmid would not result in a translatable nucleotide sequence.

Therefore an SalI/SmaI adaptor had to be used. The adaptor having 10 nucleotides was at first ligated via blunt ends with the PvuII fragment. Thereupon it was possible to form the desired construct with the protruding SalI ends of the adaptor and the pSC11-Orth vector linearized with SalI in a second step. A translatable nucleotide sequence was formed which has, however, four or five additional amino acids at the 5' end and at the 3' end of the FSME sequence. In order to confirm the orientation of the FSME fragment with respect to the vector, the DNA was analyzed with further restriction endonucleases. The translation is abandoned at stop 2 in this construct.

## Claims

1. A recombinant plasmid containing at least one replicon (oriI), at least one selection marker (sm1), at least one promoter (pr1) and DNA sequences from a pox virus genome flanking this promoter, characterized in that a multi-functional DNA sequence cassette is arranged downstream of the promoter (pr1).

2. A plasmid according to claim 1, characterized in that the multi-functional DNA sequence cassette contains multiple cloning sites (mcs).

3. A plasmid according to claim 1 or 2, characterized in that cloning sites for one or several of the following enzymes are present:

   StеI, AvrII, SalI, AccI, HindII, SpeI, NheI.

4. A plasmid according to at least one of claim 1 to 3, characterized in that the multi-functional DNA sequence cassette provides translation start and translation stop codons.

5. A plasmid according to at least one of claims 1 to 4, characterized in that the multi-functional DNA sequence cassette contains a promoter (pr2).

6. A plasmid according to at least one of claims 1 to 5, characterized in that the multi-functional DNA sequence cassette contains activating DNA sequences.

7. A plasmid according to at least one of claims 1 to 6, characterized in that the multi-functional DNA sequence cassette contains the DNA sequences for a hydrophobic membrane anchor or for signal peptides.

8. A plasmid according to at least one of claims 1 to 7, characterized in that the multi-functional DNA sequence cassette contains sequences at its 5' and/or 3' end, which are complementary to known oligonucleotide primers.

9. A plasmid according to claim 8, characterized in that the sequences are complementary to SP6 polymerase or T7 polymerase primers at the 5' and/or 3' end.

10. A plasmid according to at least one of claims 1 to 9, characterized in that the multi-functional DNA sequence cassette has the following sequence:

**5'-GCACATACGATTTAGGCCTAGGATGTCGACTAGTTAGCTAGCGTATTAATT-TCGATC-3'.**

11. A plasmid according to at least one of claims 1 to 10, characterized in that the replicon (oril) serves for the replication of the plasmid in prokaryots, preferably gram-negative bacteria.

12. A plasmid according to at least any of claims 1 to 10, characterized in that the replicon (oril) serves for the replication of the plasmid in eukaryots, preferably in yeast.

13. A plasmid according to at least cne of claims 1 to 11, characterized in that the selection marker (sm1) serves for selection in prokaryots, preferably gram-negative bacteria.

14. A plasmid according to at least one of claims 1 to 10 or 12, characterized in that the selection marker serves for selection in eukaryots, preferably yeast.

15. A plasmid according to at least one of claims 1 to 14, characterized in that the promoter (pr1) is a strong pox promoter.

16. A plasmid according to one of claims 1 to 15, characterized in that the promoter (pr1) is a promoter from the vaccinia virus.

17. A plasmid according to at least one of claims 1 to 16, characterized in that the promoter p7.5 for a polypeptide with a molecular weight of 7.5 kD is used as vaccinia promoter.

18. A plasmid according to any of claims 15 to 17, characterized in that the promoter (pr1) is a pox promoter changed by mutagenesis or a synthetic promoter.

19. A plasmid according to at least one of claims 1 to 18, characterized in that the flanking DNA sequences of the vaccinia virus correspond to non-essential regions of the virus genome.

20. A plasmid according to at least one of claims 1 to 19, characterized in that the flanking DNA sequences correspond completely or partly to the gene for the thymidine kinase.

21. A plasmid according to at least one of claims 1 to 20, characterized in that it contains a second selection marker (sm2).

**22.** A plasmid according to claim 21, characterized in that the selection marker (sm2) is the lacZ gene.

**23.** A plasmid according to claim 21, characterized in that the selection marker (sm2) is dominant.

**24.** A plasmid according to claim 23, characterized in that the selection marker (sm2) is the gene for the xanthine guanine phosporibosyl transferase.

**25.** A plasmid according to claim 23, characterized in that the selection marker (sm2) is the gene for a protein inactivating the antibiotic agent hygromycin.

**26.** A plasmid according to at least one of claims 21 to 25, characterized in that the expression of the selection marker (sm2) is under the control of a pox virus promoter.

**27.** A plasmid according to claim 26, characterized in that the promoter p11 for a polypeptide with a molecular weight of 11 kD is used as vaccinia promoter.

**28.** A plasmid according to at least one of claims 1 to 27, characterized in that the entire coding sequence of a gene is inserted in the mcs (multiple cloning site).

**29.** A plasmid according to at least one of claims 1 to 28, characterized in that coding sequences for functional regions and/or antigenic determinants of proteins are inserted.

**30.** A plasmid according to any of claims 28 or 29 characterized in that the inserted DNA is of natural and/or synthetic origin.

**31.** A plasmid accordingto at least one of claims 28 to 30, characterized in that the inserted DNA corresponds wholly or partly to the coding sequences for proteins or their derivatives from pathogens.

**32.** A plasmid according to claim 31, characterized in that the pathogens are the pathogens for pertussis, tetanus, malaria, AIDS, tick-borne encephalitis (Central European encephalitis), hepatitis B or herpes infections.

**33.** A plasmid according to at least one of claims 28 to 32, characterized in that the inserted foreign DNA codes wholly or partly for a protein having the biological function of at least one of the following proteins one of the coagulation factors II, VIII, IX, XII, XIII, von Willebrand factor, protein C and S, antithrombin III, plasminogen, hirudin, NGF, FGF, TNF, B-cell stimulating factors, proteins from the interleukine group, apolipoproteins or glycoproteins from viruses.

**34.** A plasmid according to at least one of claims 1 to 10, characterized in that it is pSC11-Orth (DMS 5734).

**35.** A process for the production of a recombinant pox virus, characterized in that a pox virus is recombined with a plasmid according to at least one of claims 1 to 34.

**36.** A process according to claim 35, characterized in that the pox virus is an attenuated virus, preferably by inactivation of the hr genes or the VGF gene.

**37.** A recombinant pox virus, characterized in that it contains a foreign gene being under the control of an additional promoter in addition to its natural DNA components and can be produced by a process according to claim 35 or 36.

**38.** A recombinant pox virus according to claim 37, characterized in that it is a vaccinia virus.

**39.** Use of the recombinant pox virus according to claim 37 or 38 for the expression of DNA sequences foreign to the pox-virus.

**40.** Use of the recombinant pox virus according to claim 39, characterized in that the DNA sequences foreign to the pox-virus code completely or partly for proteins or their derivatives from pathogens.

**41.** Use of the recombinant pox virus according to claim 40, characterized in that the pathogens can cause pertussis, tetanus, malaria, AIDS, tick borne -encephalitis, hepatitis B and herpes infections.

**42.** Use of the recombinant pox virus according to claim 39, characterized in that the DNA sequence foreign to the pox-virus code completely or partly for a protein with the biological function of at least one of the following proteins one of the coagulation factors II, VIII, IX, XII, XIII, von Willebrand factor, protein C and S, antithrombin III, plasminogen, hirudin, NGF, FGF, TNF, B-cell stimulating factors, proteins from the interleukine group, apolipoproteins or glycoproteins from viruses.

**43.** A cell culture, characterized in that it contains a recombinant pox virus according to claim 37 or 38.

**44.** A cell culture according to claim 43, characterized in that it is a $TK^-$ cell culture, preferably $TK^-$ 143 (CNCM; I-732).

**45.** A peptide or polypeptide, characterized in that it can be produced in a cell culture according to claim 43 or 44.

**46.** A pharmaceutical composition, characterized in that it contains one or several of the peptides and/or polypeptides according to claim 45.

**47.** A vaccine, characterized in that it contains one or several of the peptides and/or polypeptides according to claim 45.

**48.** A virusvaccine, characterized in that it contains at least one recombinant pox virus according to claim 37 or 38.

**49.** A diagnostic agent, characterized in that it contains one or several peptides and/or polypeptides according to claim 45.

**50.** Use of a vaccine according to claim 47 or 48 for the production of antibodies.

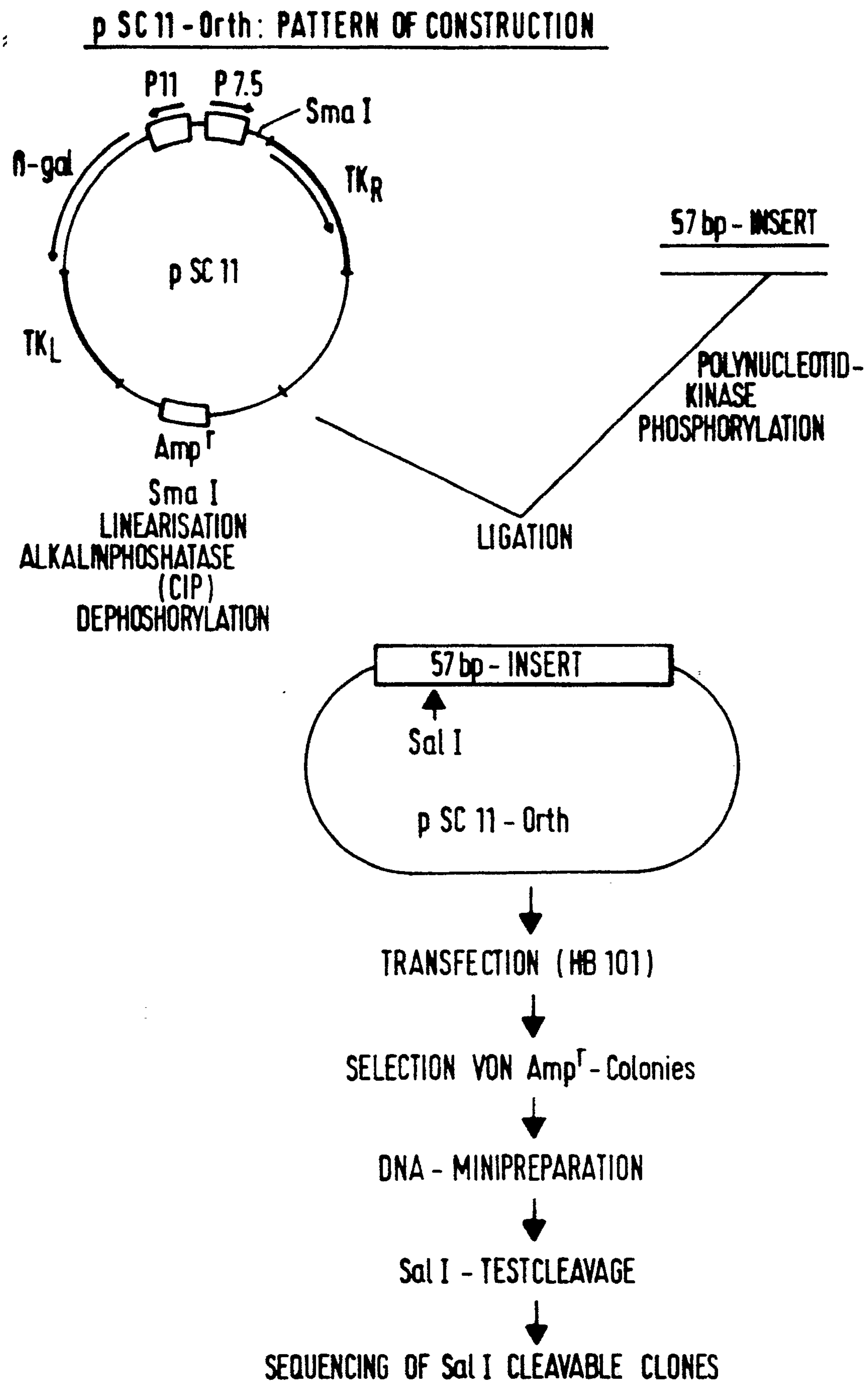
p SC 11 - Orth: PATTERN OF CONSTRUCTION
P11
P7.5
Sma I
ß-gal
TK_R
p SC 11
TK_L
Amp^r
57 bp - INSERT
POLYNUCLEOTID-
KINASE
PHOSPHORYLATION
Sma I
LINEARISATION
ALKALINPHOSHATASE
(CIP)
DEPHOSHORYLATION
LIGATION
57 bp - INSERT
Sal I
p SC 11 - Orth
TRANSFECTION (HB 101)
SELECTION VON Amp^r - Colonies
DNA - MINIPREPARATION
Sal I - TESTCLEAVAGE
SEQUENCING OF Sal I CLEAVABLE CLONES

FIG. 2

## FIG. 3

9110 Pvu II 7120 7640 7650 Bgl II

... GTA CAG CTG AAC CAA ... AAC AAT GAG TCC GAG ATC TTC pBH 10
GTC GAC CTC TAG A
Leu Asn Gln Asn Asn Glu Ser Glu

Sal I

ATG TCG ACT AGT TAG CTA GCG pSC11-Orth
TAC AGC TGA

a) Sal I UND Bgl II : BLUNT ENDS OBTAINED BY TREATMENT WITH S1-NUCLEASE

ATG CTG AAC CAA ... AAC AAT GAG TCC GAC TAG
Met Leu Asn Gln Asn Asn Glu Ser Asp

b) Sal I : BLUNT ENDS OBTAINED BY TREATMENT WITH S1-NUCLEASE

Bgl II : BLUNT ENDS OBTAINED BY FILLING-IN WITH KLENOW POLYMERASE

ATG CTG AAC CAA ... AAC AAT GAG TCC GAG ATC CTA GTT AGC TAG
Met Leu Asn Gln Asn Asn Glu Ser Glu Ile Leu Val Ser

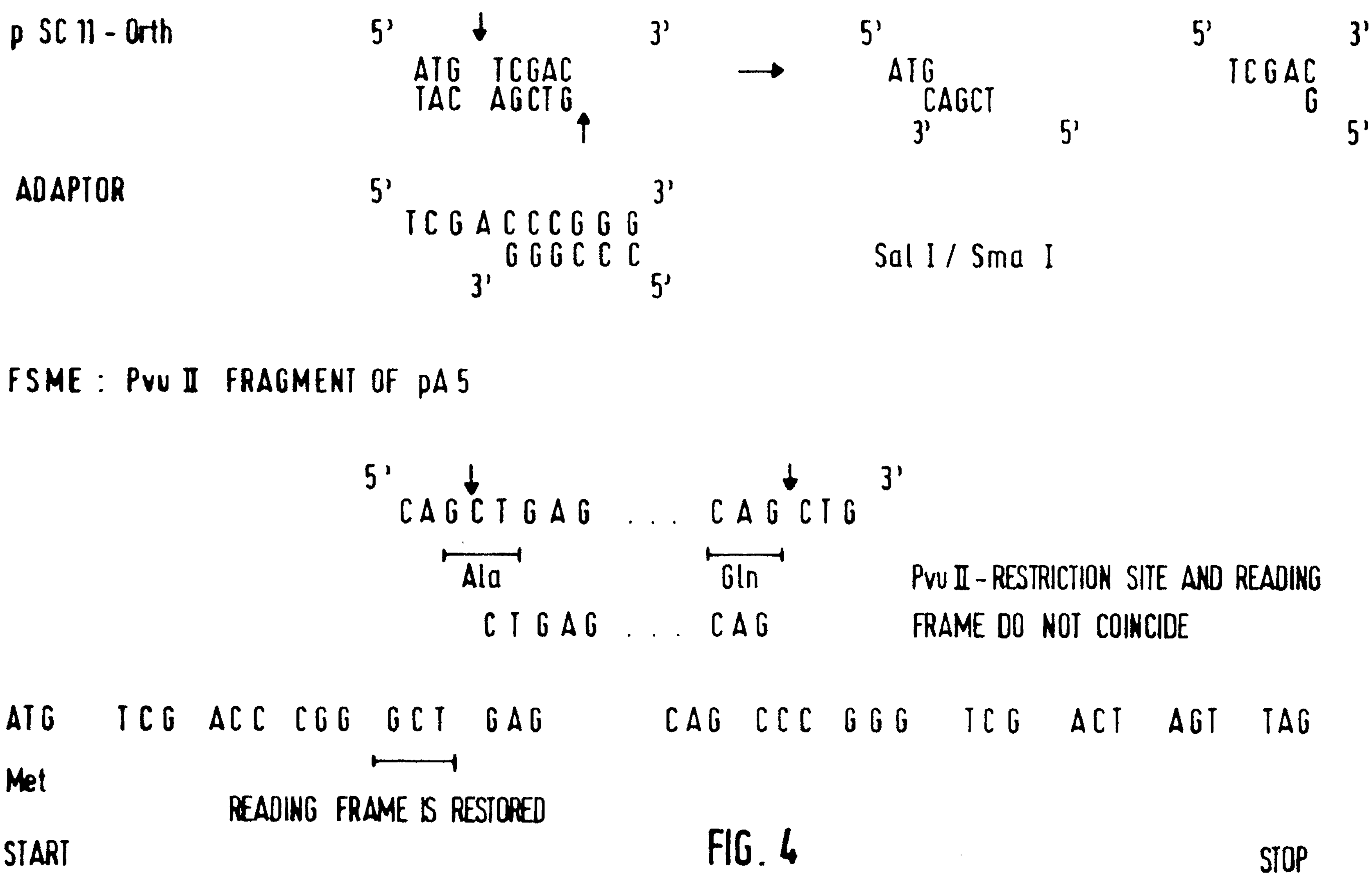
p SC 11 - Orth
5' ATG TCGAC 3'
TAC AGCTG
5' ATG CAGCT 3' 5'
5' TCGAC G 3' 5'
ADAPTOR
5' TCGACCCGGG 3'
GGGCCC
3' 5'
Sal I / Sma I
FSME : Pvu II FRAGMENT OF pA 5
5' CAGCTGAG . . . CAGCTG 3'
Ala
Gln
CTGAG . . . CAG
Pvu II - RESTRICTION SITE AND READING FRAME DO NOT COINCIDE
ATG TCG ACC CGG GCT GAG CAG CCC GGG TCG ACT AGT TAG
Met
READING FRAME IS RESTORED
START
STOP

FIG. 4

European Patent Office

# PARTIAL EUROPEAN SEARCH REPORT

which under Rule 45 of the European Patent Convention shall be considered, for the purposes of subsequent proceedings, as the European search report

Application number
EP 91 10 0773

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-89 08 716 (THE UNITED STATES OF AMERICA)<br>* The whole document, specially figure 3 *<br>-- | 1-2,4, 5,11, 13-17, 19-20, 35, 37-39, 43-49 | C 12 N 15/86<br>C 12 N 5/10<br>C 12 P 21/02 |
| X | WO-A-88 02 022 (COMMONWEALTH SCIENTIFIC AND INDUSTRIAL RESEARCH ORGANISATION)<br>* The whole document *<br>-- ./. | 1,2, 4-6, 11, 13-17, 19, 27-31, 35-49 | TECHNICAL FIELDS SEARCHED (Int. Cl.4)<br>C 12 N |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-49

Claims searched incompletely:

Claims not searched: 50

Reason for the limitation of the search:

Method for treatment of the human or animal body by surgery or therapy (see Art. 52(4) of the European Patent Convention).

| Place of search | Date of completion of the search | Examiner |
|---|---|---|
| THE HAGUE | 08-05-1991 | CHAMBONNET |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X | WO-A-89 03 879 (NATIONAL RESEARCH DEVELOPMENT CORPORATION)<br>* The whole document * | 1-2,4, 11,13, 15-17, 19-20, 28, 37-49 | |
| | -- | | |
| X | EP-A- 0 206 920 (TRANSGENE)<br>* The whole document * | 1,2,4, 11,13, 15-17, 19,20, 29,30, 33,35-39, 42,43, 45,46 | TECHNICAL FIELDS SEARCHED (Int Cl 4) |
| | -- | | |
| X | GENE, vol. 35, no. 1/2, 18th June 1985, pages 169-177, Amsterdam NL; D.B. DOXLE et al.: "Multiple-cloning-site plasmids for the rapid construction of recombinant poxviruses"<br>* The whole article * | 1,2,4, 11,13-17,19-20, 28-31, 35-49 | |
| | -- | | |
| A | EP-A-0 284 791 (IMMUNO AKTIENGESELLSCHAFT FUR CHEMISCH-MEDIZINISCHE PRODUKTE)<br>* Figure 6 * | 1-2,4-5,11, 13,15-17,19-49 | |
| | ---- | | |